# EUROPEAN PATENT APPLICATION

(11) **EP 2 486 915 A1**
(43) Date of publication of application: **15.08.2012**
(21) Application number: 12155089.1
(22) Date of filing: 13.02.2012
(51) Int. Cl.: A61K 9/06, A61K 47/10, A61K 47/20, A61K 47/32, A61K 47/38, A61K 9/00, A61K 31/192, A61K 47/14, A61K 47/26

(54) **Topical pharmaceutical compositions comprising flurbiprofen and methylsulfonylmethane**

(30) Priority: 14.02.2011 TR 201101374
(71) Applicant: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Akalin, M Nur Pehlivan, 34398 Istanbul (TR); Önder, Ramazan, 34398 Istanbul (TR)
(74) Representative: Sevinç, Erkan

(57) **Abstract**

The present invention relates to a topical pharmaceutical composition of flurbiprofen and methylsulfonylmethane comprising at least one penetration enhancer and one or more gelling agent. Furthermore, the invention relates to process for preparing the said topical pharmaceutical composition and its use for the treatment of pain and inflammatory symptoms associated with muscle-skeletal system and osteoarthritis.

## Description

### Field of Invention

The present invention relates to a topical pharmaceutical composition of flurbiprofen and methylsulfonylmethane comprising at least one penetration enhancer and one or more gelling agent. Furthermore, the invention relates to process for preparing the said topical pharmaceutical composition and its use for the treatment of pain and inflammatory symptoms associated with muscle-skeletal system and osteoarthritis.

### Background of Invention

Flurbiprofen is a well known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains. Its chemical structure is illustrated with Formula I given below.

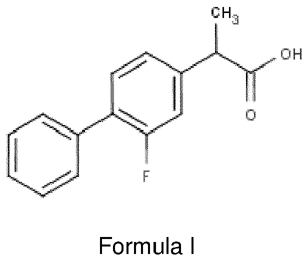

Flurbiprofen is mostly administrated orally. One disadvantage of the oral administration of flurbiprofen comprising compositions is that the patient is likely to experience unpleasant side effects, including gastrointestinal irritation. While such irritation may also result in chronic stomach upset, in some cases this can quickly manifest itself in spontaneous gastric bleeding, which can be life threatening.

Thus, the use of flurbiprofen in treating local pains and inflammations may cause a problem especially for those who have gastrointestinal system disorders. It is possible to develop various locally-administrable topical forms of flurbiprofen, in order to avoid the systemic side-effects thereof. The skin absorption rate of the relevant product to be used in topical applications, however, is quite significant. Enhancing the absorption rate provides ease of application and increases the molecule's efficiency.

Methylsulfonylmethane (MSM) is an organosulfur compound and is also known as dimethyl sulfone (DMSO2). It occurs naturally in food in a variety of fruits, vegetables and grains. MSM is commonly used for osteoarthritis, seasonal allergic rhinitis, interstitial cystitis and snoring. MSM is the primary oxidative metabolite product of dimethyl sulfoxide (DMSO).

An additional problem associated with oral pharmaceutical compositions, is that the concentration levels which must be achieved in the bloodstream must be significant in order to effectively treat distal areas of pain and inflammation. These levels are often much higher than would be necessary if it were possible to more accurately target the particular site of pain and injury. Thus there exists a need for a transdermal analgesic formulation which is capable of distal application and which has the ability to alleviate pain and inflammation in a local way.

Also in acute disorders, there arises the need of enhancing the absorption rate at the site of administration. For instance, during which local pains associated with injuries in sportive events are to be urgently alleviated, it becomes necessary to apply local anesthesia to the relevant site.

U.S. Pat. No. 6,444,234 discloses a liquid carrier composition effective for the transdermal delivery of a medicament having a given polarity, said formulation comprising (a) at least one non-aqueous non-toxic solvent; (b) limonene, lemon oil or mixture of limonene and lemon oil; (c) methylsulfonylmethane; (d) a skin stabilizer (e) a solute modifier; and (f) adenosine triphosphate (ATP) or a compound which induces generation of cyclic adenosine 3'5'monophosphate cAMP in situ or cyclic guanosine monophosphate (cGMP) in situ.

In prior art, there are also several patents which disclose NSAIDs with methylsulfonylmethane mostly in oral pharmaceutical dosage forms but none of them selected particularly flurbiprofen to combine with methylsulfonlymethane because of its poor transdermal absorption properties.

U.S. Pat. No. 6,416,772 discloses a liquid composition applied transdermally for relief of pain comprising alcohol, glycerin and an analgesic agent; the analgesic agent comprising a derivative of salicylic acid, methylsulfonylmethane and emu oil. But it is silent about the penetration problems and the use of dimethyl sulfoxide and gelling agents in combination with methylsulfonylmethane and flurbiprofen.

The compositions described above have at least one disadvantage in that the amount of drug delivered transdermally is not maximized. Accordingly, there exists need for a percutaneous delivery system for the drug flurbiprofen and methylsulfonylmethane which optimizes the delivery of them through the skin.

It is therefore an object of the present invention to provide a topical pharmaceutical composition of flurbiprofen and methylsulfonylmethane which is more effective for purposes of percutaneous delivery of them through the skin.

### Summary of the Invention

The present invention relates to an easily applicable flurbiprofen and methylsulfonylmethane topical pharmaceutical composition, which overcomes the above described problems in prior art and have additive advantages over them.

Accordingly, the main object of the present invention is to increase the rate of percutaneous penetration, thereby shortening the time period in which the active agents exert their effect.

The rate of percutaneous penetration of said combination is enhanced with the dimethyl sulfoxide and gelling agents it contains. Also surface active agents has a synergistic effect over this penetration enhancing activity of them.

Another object of the present invention is to obtain a stable topical pharmaceutical composition of flurbiprofen and methylsulfonylmethane during the shelf-life and to exhibit high safety when applied to skin.

A further object of the present invention is to obtain a formulation with local anesthetic effect, with the menthol used in said combination stimulating the receptors by which the cold sensation is perceived.

Accordingly, a topical pharmaceutical composition, more specifically a gel composition has been developed to achieve all objects referred above.

In a preferred embodiment according to the present invention, said novelty is realized with flurbiprofen and methylsulfonylmethane comprising at least one penetration enhancer and one or more gelling agent.

According to the preferred embodiment, the penetration enhancer is dimethyl sulfoxide.

In another preferred embodiment according to the present invention, the gelling agents are selected from the group comprising hydroxypropyl cellulose, hydroxyethyl cellulose, hydroxypropyl methyl cellulose, methyl cellulose, carboxymethyl cellulose, carbomer, carbomer copolymers, gelatin, aluminum monostearat, dextrin, sodium alginate, pectin, carrageanen, xanthan or mixtures thereof. Preferably the gelling agents are hydroxypropyl cellulose and carbomer.

According to the preferred embodiment, the weight ratio of hydroxypropyl cellulose and carbomer is from 10:1 to 1:10 by weight, preferably it is from 5:1 to 1:5 by weight of the total composition.

According to the preferred embodiment of the present invention the amount of dimethyl sulfoxide is from 0.5 to 30.0% by weight of the total composition, preferably it is 5.0 to 25.0% by weight of the total composition.

According to a preferred embodiment of the present invention, the topical pharmaceutical composition, further comprises surface active agents which are selected from the group comprising polysorbate, glyceryl monostearate, polyethylene glycol succinate, oleic acid, dietanolamin, sodium lauryl sulfate, propylene glycol or mixtures thereof; preferably the surface active agent is polysorbate.

According to the preferred embodiment of the present invention the amount of polysorbate is 0.05 to 5.0% by weight of the total composition, preferably it is 0.10 to 3.0% by weight of the total composition.

According to a preferred embodiment of the present invention, the topical pharmaceutical composition further comprises menthol, wherein the amount of menthol is between 0.10 to 15.0% by weight of the total composition, preferably it is 1.0 to 10.0% by weight of the total composition.

According to a preferred embodiment of the present invention, the topical pharmaceutical composition, further comprises viscosity enhancers, dissolving solvents, preservatives, antioxidants or mixtures thereof.

According to a preferred embodiment of the present invention, the topical pharmaceutical composition is in the form of gel, ointment, cream, spray or lotion, preferably it is in the form of gel.

In a further preferred embodiment of the present invention, said topical pharmaceutical composition comprise the following;

| | | |
|---|---|---|
| a. | flurbiprofen | 0.50 to 15.0 % by weight |
| b. | methylsulfonylmethane | 1.0 to 20.0 % by weight |
| c. | hydroxypropyl cellulose | 0.05 to 10.0 % by weight |
| d. | carbomer | 0.01 to 5.0 % by weight |
| e. | dimethyl sulfoxide | 0.5 to 30.0 % by weight |
| f. | polyethylene glycol | 1.0 to 50.0 % by weight |
| g. | menthol | 0.10 to 15.0 % by weight |
| h. | polysorbate | 0.05 to 5.0 % by weight |
| i. | propyl paraben | 0.001 to 2.0 % by weight |
| j. | methyl paraben | 0.01 to 2.0 % by weight |
| k. | butylated hydroxytoluene | 0.001 to 0.30 % by weight |
| l. | glycerin | 1.0 to 50.0 % by weight |
| m. | purified water | 1.0 to 50.0 % by weight |
| n. | ethyl alcohol | 1.0 to 75.0 % by weight |
| o. | NaOH / HCl | pH (5.5 ±1.0) |

Another embodiment of the present invention provides a method for preparing the topical pharmaceutical composition according to the present invention and this method comprising the steps of;
a. adding carbomer, glycerin, polyethylene glycol, dimethyl sulfoxide into purified water and swelling this mixture under stirring for 60 min. and homojenizing so as to yield the first mixture,
b. adding NaOH or HCl to the first mixture to adjust the pH and stirring,
c. adding ethyl alcohol to another container and adding flurbiprofen, methylsulfonylmethane, menthol, polysorbate, methyl paraben, propyl paraben and butylated hydroxytoluene and then adding hydroxypropyl cellulose into this mixture and stirring until they are dissolved for about 90 min., homojenizing for about 5 min. so as to give the second mixture,
d. adding the second mixture into the first mixture under stirring and then filling up the volume with ethyl alcohol and stirring 10 more min.
e. it is brought into a gelled state and continue by filling step.

According to another preferred embodiment of the present invention, the topical pharmaceutical composition is used in the treatment of pain and inflammatory symptoms associated with muscle-skeletol system and osteoarthritis.

Further advantages and embodiments of the present invention will become apparent from the following description

### Detailed Description of Invention

According to the present invention, a novel formulation with anti-inflammatory and analgesic activities is obtained, which is surprisingly rapidly absorbed and gives local anesthetic effect.

The flurbiprofen useful in accordance with this invention includes the pharmaceutically acceptable salts and esters of flurbiprofen, and further includes the conventionally used racemic mixture which comprises the S- and R- enantiomers of flurbiprofen. The topical pharmaceutical compositions of the invention comprise from 0.5 to 15.0% flurbiprofen, preferably from 1.0 to 10.0% by weight of the total composition.

The topical pharmaceutical compositions of the invention comprise from 1.0 to 20.0% methylsulfonylmethane, preferably from 5.0 to 15.0% by weight of the total composition.

The topical pharmaceutical compositions of the invention comprise from 0.5 to 30.0% dimethyl sulfoxide, preferably from 5.0 to 25.0%, more preferably from 10.0 to 20.0% by weight of the total composition. Dimethyl sulfoxide helps the composition of the present invention to improve and enhance the penetrating and spreading properties through the skin. It also helps to carry out other components easily and without damaging the membranes into biological system. These properties, surprisingly is found to have synergistic effect over flurbiprofen and methylsulfonlymethane topical composition to have better percutaneous penetration. Accordingly, dimethyl sulfoxide is used as a topical analgesic, a vehicle for topical application of pharmaceuticals, as an anti-inflammatory and an antioxidant. Other suitable penetration enhancers which can be used for the composition of the present invention may comprise 1,3-didocyl urea, 1,3-difenyl urea, 3-caren, 7-oxabicylo 2,2-heptan, ascaridol, dimetyl isosorbide, dimetyl formamide (DMF), d-Limonen, isopropyl myristat, carveol, carvon, menton, N-metyl-2-pyrolidon, NN-dimetyl toluamide, oleic acid, pinen oxide, puiegon, scylohexan oxide, siklopenten oxide, sodyum lauryl sulfate, terpinen-4-oi urea, a-pinen, a-terpineol or mixtures thereof.

The topical pharmaceutical compositions of the invention comprise from 0.01 to 15.0% gelling agents, preferably from 0.05 to 10.0% by weight. Suitable gelling agents may comprise but not limited to hydroxypropyl cellulose, hydroxyethyl cellulose, hydroxypropyl methyl cellulose, methyl cellulose, carboxymethyl cellulose, carbomer, carbomer copolymers, gelatin, aluminum monostearat, dextrin, sodium alginate, pectin, carrageanen, xanthan or mixtures thereof. Preferably the gelling agents are hydroxypropyl cellulose and carbomer. Surprisingly it is found that when the weight ratio of hydroxypropyl cellulose to carbomer is from 10:1 to 1:10 by weight, preferably from 5:1 to 1:5 by weight; it enhance the penetration properties of the formulation in combination with dimethyl sulfoxide. Accordingly, hydroxypropyl cellulose and carbomer help to obtain the desired viscosity in a stable level to enhance the penetration of the topical composition and their stabilizer and emulsifier property is also help them to show this effect easily.

Suitable surface active agents may comprise but not limited to polysorbate, glyceryl monostearat, polyethylene glycol succinate, oleic acid, diethanolamine, sodium lauril sulfate, propylene glycol or mixtures thereof. Preferably the surface active agent is polysorbate 20, polysorbate 40, polysorbate 60, polysorbate 80 or mixtures thereof. The most preferred one is polysorbate 80. The amount of polysorbate 80 is from 0.05 to 5.0%, preferably 0.1 to 3.0% by weight of the total composition. Polysorbate has also a stabilisator effect when it is used in these amounts and helps the formulation to be stable over the shelf life.

Furthermore, the topical pharmaceutical compositions of the invention comprise menthol from 0.10 to 15.0%, preferably from 1.0 to 10.0% by weight of the total composition. Menthol used in the formulation gives anesthetic effect at the side of administration as a result of stimulating the receptors by which cold sensation is perceived.

Methylsulfonylmethane and menthol provide significant relief of pain associated with mild to moderate muscle strain in adult patients. Also menthol helps to mask the bad odour of the active ingredients and other excipients used in the formulation to obtain a good patient compliance when applying to skin.

The pharmaceutical compositions according to the present invention may also comprise one or more pharmaceutically acceptable excipients. Such proper pharmaceutically acceptable excipients comprise, but are not limited to viscosity enhancers, dissolving solvents, preservatives, antioxidants or mixtures thereof.

Suitable viscosity enhancers may comprise but not limited to glycerin, pullulan, dextran, cellulose and derivatives, chitosan, carbomer or mixtures thereof. Preferably the viscosity enhancer is glycerin and the amount is from 1.0 to 50.0%, more preferably from 5.0 to 30.0% by weight of the total composition. These amounts of glycerin improve the spreading properties and minimize any balling up or drying of the compositions of the present invention when it is rubbed on the skin.

Suitable dissolving solvents may comprise but not limited to ethyl alcohol, polyethylene glycol, glycerin, isopropyl alcohol and purified water. Preferably ethyl alcohol, polyethylene glycol and purified water are used. Ethyl alcohol is also utilized as a microbiological preservative.

Suitable preservatives may comprise but not limited to methylparaben, propylparaben, sodium and potassium benzoate, imidurea, monothioglicol, phenyl mercuric derivatives, sodium sulfate, sodium meta bisulfate, potassium sorbate or mixtures thereof. Preferbly the preservatives are methylparaben and propylparaben. The amount of the preservatives is from 0.001 to 3.0%, more preferably 0.001 to 2.0% by weight of the total composition.

Suitable antioxidants may comprise but not limited to butylated hydroxyl toluene, butyl hydroxy anisole, alpha tocopherol, monothioglicol, sodium meta bisulfate, potassium meta bisulfate or mixtures thereof. Preferably the antioxidant is butylated hydroxyl toluene, The amount of the antioxidants is from 0.001 to 1.0%, preferably from 0.001 to 0.50% by weight of the total composition.

The pharmaceutical compositions according to the present invention provides spreadable, semi-solid and jelly-like gel compositions of flurbiprofen and methylsulfonylmethane. However, the topical compositions of the invention may also take the form of ointment, cream, spray or lotion.

Accordingly, the present invention may be used for treating pain and inflammatory symptoms associated with muscle-skeletol system and osteoarthritis.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

### Example

| **Content** | **amount (%) (w/w)** |
|---|---|
| Flurbiprofen | 0.50 to 15.0 |
| Methylsulfonylmethane | 1.0 to 20.0 |
| Hydroxypropyl cellulose | 0.05 to 10.0 |
| Carbomer | 0.01 to 5.0 |
| Dimethyl sulfoxide | 0.50 to 30.0 |
| Polyethylene glycol | 1.0 to 50.0 |
| Menthol | 0.10 to 15.0 |
| Polysorbate 80 | 0.05 to 5.0 |
| Propyl paraben | 0.001 to 2.0 |
| Methyl paraben | 0.01 to 2.0 |
| Butylated hydroxytoluene | 0.01 to 0.30 |
| Glycerin | 1.0 to 50.0 |
| Purified water | 1.0 to 50.0 |
| Ethyl alcohol | 1.0 to 75.0 |
| NaOH / HCL | pH 5.5 ±1.0 |

Carbomer, glycerin, polyethylene glycol and dimethyl sulfoxide is added into purified water under stirring and the mixture is swollen by keeping it stirred for about 60 min and homogenised. Thus, the first mixture is obtained, and the pH is adjusted with NaOH or HCL. Flurbiprofen, methylsulfonylmethane, menthol, polysorbate 80, methyl paraben, propyl paraben and butylated hydroxytoluene is dissolved in a separate container in ethyl alcohol. Thus, the second mixture is obtained. Then hydroxypropyl cellulose is added into the second mixture under stirring for about 90 min and then homogenized for 5 more min. The second mixture is added to the first mixture under stirring for about 10 min and filled up with ethyl alcohol. Then, it is brought into a gelled state and the procedure is completed and continue by filling step.

## Claims

1. A topical pharmaceutical composition of flurbiprofen and methylsulfonylmethane comprising dimethyl sulfoxide and one or more gelling agent wherein the gelling agents are selected from the group comprising hydroxypropyl cellulose, hydroxyethyl cellulose, hydroxypropyl methyl cellulose, methyl cellulose, carboxymethyl cellulose, carbomer, carbomer copolymers, gelatin, aluminum monostearat, dextrin, sodium alginate, pectin, carrageanen, xanthan or mixtures thereof.

2. The topical pharmaceutical composition according to claim 1, wherein the gelling agents are preferably hydroxypropyl cellulose and carbomer.

3. The topical pharmaceutical composition according to claim 2, wherein the weight ratio of hydroxypropyl cellulose to carbomer is from 10:1 to 1:10 by weight, preferably it is from 5:1 to 1:5 by weight of the total composition.

4. The topical pharmaceutical composition according to claim 1, wherein the amount of dimethyl sulfoxide is from 0.50 to 30.0 % by weight of the total composition, preferably it is 5.0 to 25.0 % by weight of the total composition.

5. The topical pharmaceutical composition according to any of the preceding claims, further comprising surface active agents.

6. The topical pharmaceutical composition according to claim 5, wherein the surface active agent is selected form the group comprising, polysorbate, glyceryl monostearate, polyethylene glycol succinate, oleic acid, dietanolamin, sodium lauryl sulfate, propylene glycol or mixtures thereof; preferably the surface active agent is polysorbate.

7. The topical pharmaceutical composition according to claim 6, wherein the amount of polysorbate is 0.05 to 5.0 % by weight of the total composition, preferably it is 0.10 to 3.0 % by weight of the total composition.

8. The topical pharmaceutical composition according to any of the preceding claims, further comprising menthol.

9. The topical pharmaceutical composition according to claim 8, wherein the amount of menthol is between 0.10 to 15.0 % by weight of the total composition, preferably it is 1.0 to 10.0 % by weight of the total composition.

10. The topical pharmaceutical composition according to any of the preceding claims, further comprising viscosity enhancers, dissolving solvents, preservatives, antioxidants or mixtures thereof.

11. The topical pharmaceutical composition according to any of the preceding claims, wherein it is in the form of gel, ointment, cream, spray or lotion, preferably it is in the form of gel.

12. The topical pharmaceutical composition according to any of the preceding claims, comprising,
| | | |
|---|---|---|
| a. | flurbiprofen | 0.50 to 15.0 % by weight |
| b. | methylsulfonylmethane | 1.0 to 20.0 % by weight |
| c. | hydroxypropyl cellulose | 0.05 to 10.0 % by weight |
| d. | carbomer | 0.01 to 5.0 % by weight |
| e. | dimethyl sulfoxide | 0.5 to 30.0 % by weight |
| f. | polyethylene glycol | 1.0 to 50.0 % by weight |
| g. | menthol | 0.10 to 15.0 % by weight |
| h. | polysorbate | 0.05 to 5.0 % by weight |
| i. | propyl paraben | 0.001 to 2.0 % by weight |
| j. | methyl paraben | 0.01 to 2.0 % by weight |
| k. | butylated hydroxytoluene | 0.001 to 0.30 % by weight |
| l. | glycerin | 1.0 to 50.0 % by weight |
| m. | purified water | 1.0 to 50.0 % by weight |
| n. | ethyl alcohol | 1.0 to 75.0 % by weight |
| o. | NaOH or HCl | pH (5.5 ±1.0) |

13. Process for preparing the topical pharmaceutical composition according to claim 12, comprising the steps of;
a. Adding carbomer, glycerin, polyethylene glycol, dimethyl sulfoxide into purified water and swelling this mixture under stirring for 60 min. and homojenizing so as to yield the first mixture,
b. adding NaOH or HCl to the first mixture to adjust the pH and stirring,
c. adding ethyl alcohol to another container and adding flurbiprofen, methylsulfonylmethane, menthol, polysorbate, methyl paraben, propyl paraben and butylated hydroxytoluene and then adding hydroxypropyl cellulose into this mixture and stirring until they are dissolved for about 90 min., homojenizing for about 5 min. so as to give the second mixture,
d. adding the second mixture into the first mixture under stirring and then filling up the volume with ethyl alcohol and stirring 10 more min.
e. it is brought into a gelled state and continue by filling step.

14. The topical pharmaceutical composition according to any previous claims, for use in the treatment of pain and inflammatory symptoms associated with muscle-skeletal system and osteoarthritis.
